# EUROPEAN PATENT SPECIFICATION

(11) **EP 1 612 222 B1**
(45) Date of publication and mention of the grant of the patent: **14.07.2010**
(21) Application number: 05011523.7
(22) Date of filing: 21.09.2001
(51) Int. Cl.: C07K 14/72

(54) **Olfactory and pheromones G-protein coupled receptors**
G-Protein Rezeptoren für Geruchs- und Pheromonmoleküle
Récepteurs olfactifs et de phéromones couplés à la protéine G

(30) Priority: 22.09.2000 EP 00870211
(43) Date of publication of application: 04.01.2006
(62) Divisional of application: 01971527.5
(73) Proprietor: ChemCom S.A., 1070 Brussels (BE)
(72) Inventor: Veithen, Alex, 1400 Nivelles (BE)
(74) Representative: De Clercq, Ann G. Y.

(56) References cited:
- EP-A- 1 270 724
- WO-A-00/21985
- WO-A-00/35274
- WO-A-02/16548
- WO-A-97/14790
- WO-A-99/00422
- GLUSMAN G ET AL: "Sequence, Structure, and Evolution of a Complete Human Olfactory Receptor Gene Cluster" GENOMICS, ACADEMIC PRESS, SAN DIEGO, US, vol. 63, no. 2, 15 January 2000 (2000-01-15), pages 227-245, XP004439442 ISSN: 0888-7543
- KRAUTWURST D ET AL: "Identification of ligands for olfactory receptors by functional expression of a receptor library" CELL, CELL PRESS, CAMBRIDGE, NA, US, vol. 95, 25 June 1998 (1998-06-25), pages 917-926, XP002153217 ISSN: 0092-8674
- DATABASE EMBL [Online] 1 February 1999 (1999-02-01), "Homo sapiens PAC clone RP5-905H7 from 7, complete sequence." XP002381787 retrieved from EBI accession no. EM_HUM:AC006455 Database accession no. AC006455
- RODRIGUEZ I.; MOMBAERTS P.: 'Novel human vomeronasal receptor-like genes reveal species-specific families.' CURRENT BIOLOGY vol. 12, no. 12, 2002, pages R409 - R411
- BOSCHAT C. ET AL: 'Pheromone detection mediated by a V1r vomeronasal receptor.' NATURE NEUROSCIENCE vol. 5, no. 12, 18 November 2002, pages 1261 - 1262

## Description

### Field of the invention

The present specification is related to newly identified, isolated and purified members of the family of olfactory ligand (odorant or any other molecule able to interact with said receptor) and the family of pheromone ligand G-protein-coupled (preferably human) receptors as well as to the various uses that can be made of said receptors.

The specification is also related to the polynucleotides sequences encoding said (preferably human) receptors.

The specification is further related to methods using receptor polypeptides and polynucleotides applicable to diagnostic and treatment in receptor-mediated disorders.

The specification is further related to ligand-screening methods using the receptor polypeptides and polynucleotides, to identify agonists and antagonists used as improved flavours or perfumes, as well as the prevention and/or treatment of various disorders.

The specification further encompasses agonists and antagonists based on the said olfactory and pheromones receptor polypeptides and polynucleotides and biosensors comprising said receptor polypeptides.

The specification is further related to procedures for producing the receptor polypeptides and polynucleotides as described herein, preferably by genetic recombinant methods.

### Background of the invention

G-protein coupled receptors (GPCRs) are proteins responsible for transducing a signal within a cell. GPCRs have usually seven transmembrane domains. Upon binding of a ligand to an extra-cellular portion or fragment of a GPCR, a signal is transduced within the cell that results in a change in a biological or physiological property or behaviour of the cell. GPCRs, along with G-proteins and effectors (intracellular enzymes and membrane channels modulated by G-proteins), are the components of a modular signalling system that connects the state of intra-cellular second messengers to extra-cellular inputs.

GPCR genes and gene products are potential causative agents of disease and these receptors seem to be of critical importance to both the central nervous system and peripheral physiological processes.

The GPCR protein superfamily is represented in five families : Family I, receptors typified by rhodopsin and the beta2-adrenergic receptor and currently represented by over 200 unique members; Family II, the parathyroid hormone/calcitonin/secretin receptor family; Family III, the metabotropic glutamate receptor family, Family IV, the CAMP receptor family, important in the chemotaxis and development of *D. discoideum;* and Family V, the fungal mating pheromone receptor such as STE2.

G proteins represent a family of heterotrimeric proteins composed of α, β and γ subunits, that bind guanine nucleotides. These proteins are usually linked to cell surface receptors (receptors containing seven transmembrane domains).

Following ligand binding to the GPCR, a conformational change is transmitted to the G protein, which caused the α-subunit to exchange a bound GDP molecule for a GTP molecule and to dissociate from the βγ-subunits.

The GTP-bound form of the α, β and γ-subunits typically functions as an effector-modulating moiety, leading to the production of second messengers, such as cAMP (e.g. by activation of adenyl cyclase), diacylglycerol or inositol phosphates.

Greater than 20 different types of α-subunits are known in humans. These subunits associate with a small pool of β and γ subunits. Examples of mammalian G proteins include Gi, Go, Gq, Gs and Gt. G proteins are described extensively in Lodish et al., Molecular Cell Biology, (Scientific American Books Inc., New York, N.Y., 1995).

Known and unknown GPCRs constitute now major targets for drug action and development.

More than 300 GPCRs have been cloned thus far and it is generally assumed that it exists well over 1000 such receptors. Mechanistically, approximately 50-60% of all clinically relevant drugs act by modulating the functions of various GPCRs (Cudermann et al., J. Mol. Med., Vol. 73, pages 51-63, 1995).

### Summary of the invention

The present invention provides any one and each of (i) to (xii) as set out here below:
(i) a pheromone G-protein coupled receptor having an amino acid sequence with a sequence identity higher than 75%, 80%, 85%, 90% or 95% as represented in SEQ ID NO 4;
(ii) a G-protein coupled receptor as set forth in (i) above having the amino acid sequence as represented in SEQ ID NO 4 or a specific active portion thereof, wherein said portion is a partial or deleted receptor which comprises modifications or deletions of the complete amino acid sequence and which still maintains the active site(s) necessary for the binding of a ligand able to interact with said receptor;
(iii) a polynucleotide encoding the amino acid sequences of the G-protein coupled receptor as set forth in (i) or (ii) above;
(iv) a polynucleotide as set forth in (iii) above having the nucleotide sequence as depicted in SEQ ID NO 3;
(v) an antibody or specific hypervariable regions thereof that binds specifically to the receptor as set forth in (i) or (ii) above;
(vi) an antibody as set forth in (v) above which is a monoclonal antibody;
(vii) a vector comprising the polynucleotide as set forth in (iii) or (iv) above;
(viii) a cell transformed by the vector as set forth in (vii) above;
(ix) a non-human mammal comprising a partial or total deletion of the polynucleotide as set forth in (iii) or (iv) above encoding a receptor as set forth in (i) or (ii) above, preferably an non-human mammal comprising an homologous recombination "knock-out" of said polynucleotide or a transgenic non-human mammal overexpressing above natural level said polynucleotide;
(x) a method for the screening (detection and possibly recovering) of compounds which are agonists, antagonists or inhibitors of natural compounds to the receptor as set forth in (i) or (ii) above, said method comprising:
   - contacting a cell or cell extract from the cell transfected with a vector as set forth in (vii) above,
   - possibly isolating a membrane fraction from the cell extract or the complete cell with a natural compound binding to said receptor under conditions permitting binding of said compound to said receptor, possibly by the activation of a functional response, and
   - detecting the presence of any such compound by means of a bioassay, preferably a modification in the production of a second messenger or an increase in the receptor activity in the presence of another molecule (or compound) working as an agonist, antagonist or inhibitor of a natural known compound to the receptor and thereby recovering and determining whether said molecule (or other compound) is able to work as an agonist, antagonist or inhibitor of the natural compound to its receptor;
(xi) use of the receptor as set forth in (i) or (ii) above or the polynucleotide as set forth in (iii) or (iv) above for the preparation of a medicament for the improvement, elimination or substitution of an existing taste and/or a fragrance of (or in) food and/or cosmetical products;
(xii) a biosensor or a technical device comprising a receptor as set forth in (i) or (ii) above.

The present specification is further related to newly isolated and purified members of olfactory (odorant/ligand) and pheromones/ligands (SEQ ID NO. 1 to SEQ.ID NO. 548) and G-protein-coupled receptors as well as to polynucleotide sequences including recombinant sequences encoding said receptors, described hereafter.

The present specification is also related to non-described nucleotide and/or amino acid sequences homologous to the sequences corresponding to the receptors described hereafter.

Homologous sequences mean sequences which present a high sequence identity (which present an identity higher than 75%, 80%, 85%, 90% or 95%) with the complete sequence described hereafter.

Also described in the present specification is a specific active portion of said sequences or a library of said active portion (ligand binding) of these sequences. Said portions could be partial or deleted receptors which comprise modifications (punctual mutations) or deletions upon the complete nucleotide or amino acid sequences and which still maintain the active site(s) necessary for the binding of a specific ligand able to interact with said receptors.

Homologous sequences of the sequences described herein may comprise similar receptors which exist in other animal (vertebrates, preferably mammalian) or specific human populations, but which are involved in the same biochemical pathway.

Such homologous sequences may comprise addition, deletion or substitution of one or more amino acids or nucleotides, which does not substantially alter the functional characteristics of the receptor(s) according to the invention in order to form preferably an hybrid polypeptide with another transmembrane protein, suitable for rapid ligand screening.

Thus, the specification encompasses also a receptor and corresponding nucleotide sequence having exactly the same amino acid or nucleotide sequences (derived from olfactory neurons or olfactory epithelium) as shown in the enclosed sequence listing, as well as molecules which differ, but which are not retaining the basic qualitative binding properties of the receptors described herein.

The specification is preferably related to said human receptors characterised by the complete nucleotide and amino acid sequences described hereafter, agonist and antagonist compounds or inhibitors, preferably antibodies or specific hypervariable portions thereof that bind specifically to said receptors (i.e. that have at least a 10 fold greater affinity for said receptors than any other naturally occurring antibody) and specifically antibodies made by a process involving the injection of a pharmaceutically acceptable preparation of such amino acid sequence into a animal capable of producing antibodies directed against said receptor.

For instance, a monoclonal antibody to the receptor according to the invention is obtained by injecting of an expression plasmid comprising the DNA encoding said receptor into a non human mammal and than fusing mouse spleen cells with myeloma cells.

The present invention is also related to the polynucleotide according to the invention, possibly linked to other expression sequences and incorporated into a vector and host cells transformed by such vector (plasmid, virus such as an adenovirus, liposomes, cationic vesicles,...).

The present invention is also related to the recombinant, preferably human receptor according to the invention, produced by such host cells according to the method well known by the person skilled in the art.

The present invention is also related to a transgenic non human mammal comprising a partial or total deletion of the genetic sequences encoding the receptor according to the invention, preferably a non human mammal comprising an homologous recombination "knock-out" of the nucleotide sequences (polynucleotides) according to the invention or a transgenic non human mammal overexpressing above natural level said nucleotide sequences (polynucleotides).

Said transgenic non human mammal can be obtained by methods well known by the person skilled in the art, for instance by the one described in the document WO98/20112 using classical techniques based upon the transfection of embryonic stem cells, preferably according to the method described by Carmeliet et al., Nature, Vol. 380, p. 435-439, 1996.

Preferably, said transgenic non human mammal overexpressing the polynucleotides according to the invention or portions thereof comprises said polynucleotides or active portions thereof incorporated in a DNA construct with an inducible promoter allowing its overexpression and possibly tissues and other specific regulatory elements.

Another aspect of the present invention is related to a method for the screening (detection and possibly recovering) of (odorant, pheromones or inhibitor thereof) compounds which are known or not known to be agonists, antagonists or inhibitors of natural compounds to the receptor according to the invention, said method comprising :
- contacting a cell or cell extract from the cell transfected with a vector expressing the polynucleotides encoding the receptor(s) according to the invention or active portion(s) thereof,
- possibly isolating a membrane fraction from the cell extract or the complete cell with a natural compound binding to said receptor under conditions permitting binding of said compound to said receptor, possibly by the activation of a functional response (resulting in a detectable signal) and
- detecting the presence of any such compound by means of a bioassay, preferably a modification in the production of a second messenger or an increase in the receptor activity in the presence of another molecule (or compound) working as an agonist, antagonist or inhibitor of a natural known compound to the receptor according to the invention and thereby possibly recovering and determining whether said molecule (or other compound) is able to work as an agonist, antagonist or inhibitor of the natural compound to its receptor, preferably the second messenger assay comprises the measurement of intra-cellular cAMP, intracellular inositol phosphates, intra-cellular diacylglycerol concentration, arachinoid acid concentration, MAP kinase(s) or tyrosine kinase(s) pathways or intra-cellular calcium mobilisation.

The present specification is also related to said molecules or compounds, preferably pheromones or flavours, including possible toxic molecules identified by said screening (identification and recovering) method and to their pharmaceutical, cosmetical and industrial (production of detergents, soap, shampoo, fragrances, odorant fingerprints, appetite surpressant compounds, etc.) use.

Such molecules or compounds may be used also for obtaining in mammal a modification of its taste and its physiological reactions to odorant pheromones or flavours.

The present specification is also related to a (preferably nasal) spray for controlling appetite comprising the identified compounds or molecules by the method according to the invention in a suitable carrier.

Another application of such receptor is the trapping of odour by using the receptor, the cell or membrane according to the invention wherein the desired odour ligand is absorbed by the binding of the odorant ligand to the odorant receptor.

The present invention is further related to an odour trap, using said method for trapping odour.

Preferably, the tested odorants or pheromones upon the receptor according to the invention are advantageously selected from different body secretions such as sweat, salive, urine, vaginal secretions, sperm, etc. The tested odorants or pheromones upon the receptor are also advantageously selected from the group of 16-androstene family, such as 5α-androst-16-en-3α-ol, 5α-androst-16-en-3-one, androstadienone, a human pheromone described previously (Grosser et al., Psychoneuroendocrinology vol. 25, pages 289-299, 2000) and other androstenol derivatives; the group consisting of estrene family, such as 1,3,5(10),16-estratetraen-3-ol and other estradiol derivatives described in PCT/US92/00219, PCT/US92/00220 and EP0562843 ; the group consisting of progestin family such as the human pheromone pregna-4,20-diene-3,6-dione (Monti-Bloch et al., J. Steroid Biochem. Mol. Biol. Vol. 65, pages 237-242) and other progesterone derivatives , the group consisting of small fatty acids such as acetic acid, propionic acid, butyric acid, isovaleric acid and isocaproic acid that compose the putative human vaginal pheromone copulin and such as the trans-3-methyl-2-hexanoic acid found in human sweat, cyclic organic compounds homologous to known animal pheromone such as dehydro-exo-brevicomin and such as nepetalactone, and human homologs of the murine protein aphrodisin.

Other examples of such compounds are molecules present in vapour emanating from narcotics, like cocaine, marihuana, heroin, hashish, angel dust, gasoline, natural gas, alcohol, decayed human flesh, explosives, plastic explosives, fire arms, gun powder, toxic fumes, noxious fumes or dangerous fumes, etc.

Said molecules or compounds could be used also for promoting or suppressing chemical communication. Every stimuli involving the emission by an organism or an exogenous source of chemical compounds able to modify the probability of response of another organism or a part of another organism.

These molecules or compounds could be used in the treatment or prevention of various disorders affecting, for instance, cell migration, cell death, cell growth, psychotic and neurological disorders, including anxiety, schizophrenia, maniac depression, depression or mound modification, etc.

These molecules or compounds could be used also for improving contraceptive medication, treatment promoting axonal growth, neural connection and nerves regeneration, in modulating male and female endocrine functions, or may effect the menstrual cycle. Indeed, it is known that several of said receptors can be present at the surface of spermatozoids and can then find contraceptive application or could be used in the treatment/prevention of sterility/fertility. These receptors are also present in various neurolfactory neurons and can improve their connection, especially upon the olfactory bulb or upon other tissue comprising said olfactory receptors.

They could be also used for the treatment or the prevention of various animal or human behaviours, such as stimulation and/or suppression of appetite, stimulation and/or suppression of motivation and sexual attraction, stimulation and/or suppression of aggressiveness, stimulation and/or suppression of alarm and defence behaviours, stimulation and/or suppression of territory and trail-marking, stimulation and/or suppression of social regulation and recognition, stimulation and/or suppression of mother-child recognition, etc.

The screening method according to the invention could be performed by well known methods to the person skilled in the art, preferably high-throughput screening, diagnostic and dosage devices based upon the method described in the International patent application WO00/02045 performed upon various solid supports such as micro-titer plates or biochips according to known techniques by the person skilled in the art.

The present specification is also related to the molecules characterized and possibly recovered by said method, including the pharmaceutical composition comprising a sufficient amount of said molecules and a pharmaceutically acceptable carrier for the preparation of a medicament in the prevention and/or the treatment of specific diseases.

A last aspect of the present invention is related to a biosensor or any technical device comprising the receptors according to the invention for the detection of the specific above-mentioned compounds or molecules.

## Claims

1. A pheromone G-protein coupled receptor having an amino acid sequence with a sequence identity higher than 75%, 80%, 85%, 90% or 95% as represented in SEQ ID NO 4.

2. A G-protein coupled receptor according to claim 1 having the amino acid sequence as represented in SEQ ID NO 4 or a specific active portion thereof, wherein said portion is a partial or deleted receptor which comprises modifications of or deletions from the complete amino acid sequence and which still maintains the active site(s) necessary for the binding of a ligand able to interact with said receptor.

3. A polynucleotide encoding the amino acid sequences of the G-protein coupled receptor according to claim 1 or 2.

4. A polynucleotide according to claim 3 having the nucleotide sequence as depicted in SEQ ID NO 3.

5. An antibody or specific hypervariable regions thereof that binds specifically to the receptor according to claim 1 or 2.

6. An antibody according to claim 5 which is a monoclonal antibody.

7. A vector comprising the polynucleotide according to claim 3 or 4.

8. A cell transformed by the vector according to claim 7.

9. A non-human mammal comprising a partial or total deletion of the polynucleotide according to claim 3 or 4 encoding a receptor according to claim 1 or 2, preferably an non-human mammal comprising an homologous recombination "knock-out" of said polynucleotide or a transgenic non-human mammal overexpressing above natural level said polynucleotide.

10. A method for the screening (detection and possibly recovering) of compounds which are agonists, antagonists or inhibitors of natural compounds to the receptor according to claim 1 or 2, said method comprising:
- contacting a cell or cell extract from the cell transfected with a vector according to claim 7,
- possibly isolating a membrane fraction from the cell extract or the complete cell with a natural compound binding to said receptor under conditions permitting binding of said compound to said receptor, possibly by the activation of a functional response, and
- detecting the presence of any such compound by means of a bioassay, preferably a modification in the production of a second messenger or an increase in the receptor activity in the presence of another molecule (or compound) working as an agonist, antagonist or inhibitor of a natural known compound to the receptor and thereby recovering and determining whether said molecule (or other compound) is able to work as an agonist, antagonist or inhibitor of the natural compound to its receptor.

11. Use of the receptor according to claim 1 or 2 or the polynucleotide according to claim 3 or 4 for the preparation of a medicament for the improvement, elimination or substitution of an existing taste and/or a fragrance of (or in) food and/or cosmetical products.

12. A biosensor or a technical device comprising a receptor according to claim 1 or 2.

## Patentansprüche

1. G-Protein-gekoppelter Pheromonrezeptor, der eine Aminosäuresequenz mit einer Sequenzidentität zu der in SEQ-ID Nr. 4 dargestellten Sequenz aufweist, die höher ist als 75 %, 80 %, 85 %, 90 % oder 95 %.

2. G-Protein-gekoppelter Rezeptor nach Anspruch 1, der eine Aminosäuresequenz, wie sie in SEQ-ID Nr. 4 dargestellt ist, oder einen spezifischen, aktiven Teilbereich davon aufweist, wobei der Teilbereich ein partieller oder ein deletierter Rezeptor ist, der Modifikationen oder Deletionen der vollständigen Aminosäuresequenz umfasst und der trotzdem die aktive(n) Stelle(n) vorhält, die zur Bindung von einem Liganden, der zur Wechselwirkung mit dem Rezeptor in der Lage ist, notwendig ist/sind.

3. Polynukleotid, das die Aminosäuresequenzen von dem G-Protein-gekoppelten Rezeptor nach Anspruch 1 oder 2 kodiert.

4. Polynukleotid nach Anspruch 3, das die in SEQ-ID Nr. 3 dargestellte Nukleotidsequenz aufweist.

5. Antikörper oder spezifische hypervariable Regionen davon, der/die spezifisch an den Rezeptor nach Anspruch 1 oder 2 bindet/binden.

6. Antikörper nach Anspruch 5, der ein monoklonaler Antikörper ist.

7. Vektor, der das Polynukleotid nach Anspruch 3 oder 4 umfasst.

8. Zelle, die mit dem Vektor nach Anspruch 7 transformiert ist.

9. Nicht-menschliches Säugetier, das eine teilweise oder eine vollständige Deletion von dem Polynukleotid nach Anspruch 3 oder 4, das einen Rezeptor nach Anspruch 1 oder 2 kodiert, umfasst, vorzugsweise ein nicht-menschliches Säugetier, das ein mittels homologer Rekombination erzeugtes "Knock-out" des Polynukleotids umfasst oder ein transgenes nicht-menschliches Säugetier, welches das Polynukleotid über das natürliche Niveau hinaus überexprimiert.

10. Verfahren zum Überprüfen (Nachweis und gegebenenfalls Entdecken) von Verbindungen, die als Agonisten, Antagonisten oder Hemmstoffe von natürlichen Verbindungen an dem Rezeptor nach Anspruch 1 oder 2 wirken, wobei das Verfahren umfasst:
- Inkontaktbringen mit einer Zelle oder einem Zellextrakt von der Zelle, die mit einem Vektor nach Anspruch 7 transfiziert ist,
- gegebenenfalls Isolieren einer Membranfraktion aus dem Zellextrakt oder der vollständigen Zelle mit einer natürlichen Verbindung, die an den Rezeptor unter Bedingungen bindet, die eine Bindung von der Verbindung an den Rezeptor gestatten, gegebenenfalls durch die Aktivierung von einer funktionellen Reaktion, und
- Nachweisen des Vorhandenseins von irgendeiner derartigen Verbindung mit Hilfe von einem Bioassay, vorzugsweise durch eine Veränderung in der Produktion von einem Second Messenger oder durch eine Zunahme in der Rezeptoraktivität in der Gegenwart von einem weiteren Molekül (oder einer weiteren Verbindung), die als Agonist, Antagonist oder Inhibitor von einer bekannten natürlichen Verbindung an dem Rezeptor wirken und **dadurch** Entdecken und Bestimmen, ob das Molekül (oder die andere Verbindung) fähig ist, als ein Agonist, Antagonist oder Inhibitor von der natürlichen Verbindung an dem Rezeptor zu wirken.

11. Verwendung von dem Rezeptor nach Anspruch 1 oder 2 oder dem Polynukleotid nach Anspruch 3 oder 4 für die Herstellung von einem Medikament für die Verbesserung, Beseitigung oder den Ersatz von einem vorhandenen Geschmack und/oder einem Duft von (oder in) Lebensmitteln und/oder kosmetischen Produkten.

12. Biosensor oder technische Vorrichtung, die einen Rezeptor nach Anspruch 1 oder 2 umfasst.

## Revendications

1. Récepteur des phéromones couplé à la protéine G ayant une séquence d'acides aminés présentant une identité de séquence supérieure à 75 %, 80 %, 85 %, 90 % ou 95 % telle que représentée dans SEQ ID NO : 4.

2. Récepteur des phéromones couplé à la protéine G selon la revendication 1, ayant la séquence d'acides aminés telle que représentée dans SEQ ID NO : 4 ou une partie active spécifique de celle-ci, ladite partie étant un récepteur partiel ou délété qui comprend des modifications de ou des délétions dans la séquence d'acides aminés complète et qui conserve toujours le(s) site(s) actif(s) nécessaire(s) pour la liaison d'un liant capable d'interagir avec ledit récepteur.

3. Polynucléotide codant pour les séquences d'acides aminés du récepteur couplé à la protéine G selon la revendication 1 ou 2.

4. Polynucléotide selon la revendication 3, ayant la séquence de nucléotides telle que représentée dans SEQ ID NO : 3.

5. Anticorps ou régions hypervariables spécifiques de celui-ci, qui se lie spécifiquement au récepteur selon la revendication 1 ou 2.

6. Anticorps selon la revendication 5, qui est un anticorps monoclonal.

7. Vecteur comprenant le polynucléotide selon la revendication 3 ou 4.

8. Cellule transformée par le vecteur selon la revendication 7.

9. Mammifère non humain comprenant une délétion partielle ou totale du polynucléotide selon la revendication 3 ou 4, codant pour un récepteur selon la revendication 1 ou 2, de préférence un mammifère non humain qui comprend un « knock-out » de recombinaison homologue dudit polynucléotide ou un mammifère non humain transgénique sur-exprimant au-dessus du niveau naturel ledit polynucléotide.

10. Procédé de criblage (détection et éventuellement récupération) de composés qui sont des agonistes, des antagonistes ou des inhibiteurs de composés naturels du récepteur selon la revendication 1 ou 2, ledit procédé comprenant :
- la mise en contact d'une cellule ou d'un extrait cellulaire de la cellule transfectée avec un vecteur selon la revendication 7,
- l'isolement facultatif d'une fraction de membrane de l'extrait cellulaire ou de la cellule entière avec un composé naturel se liant au dit récepteur dans des conditions permettant la liaison dudit composé au dit récepteur, facultativement par l'activation d'une réponse fonctionnelle, et
- la détection de la présence de tout composé de ce genre au moyen d'un biodosage, de préférence d'une modification de la production d'un second messager ou d'une augmentation de l'activité du récepteur en présence d'une autre molécule (ou composé) fonctionnant comme un agoniste, un antagoniste ou un inhibiteur d'un composé naturel connu du récepteur et ainsi la récupération et la détermination du fait que ladite molécule (ou autre composé) est ou non capable de fonctionner comme un agoniste, un antagoniste ou un inhibiteur du composé naturel pour son récepteur.

11. Utilisation du récepteur selon la revendication 1 ou 2, ou du polynucléotide selon la revendication 3 ou 4, pour la préparation d'un médicament destiné à l'amélioration, à l'élimination ou à la substitution d'un goût existant et/ou d'un parfum de (ou dans des) produits alimentaires et/ou cosmétiques.

12. Biocapteur ou dispositif technique qui comprend un récepteur selon la revendication 1 ou 2.


### SEQUENCE LISTING

<110> ChemCom S.A.
   Veithen, Alex
<120> Olfactory and Pheromones G-Protein coupled Receptors
<130> P.CHEM.01/WO
<160> 548
<170> PatentIn version 3.1
<210> 3
   <211> 1074
   <212> DNA
   <213> Homo Sapiens
<220>
   <221> CDS
   <222> (1)..(1071)
   <223>
<400> 3
   atg ttg aaa ttg gtt att att gag aac atg gca gaa att atg cta ttc      48
   tca tta gat ctc ttg ctt ttc tcc aca gat atc ctt tgc ttt aat ttt      96
   cct tct aag atg atc aaa ctt cct ggt ttt att acc ata caa atc ttc     144
   ttt tat cca caa gcc agc ttt gga att tca gca aac acc atc ctt ctt     192
   ctt tac cac atc ttc acc ttt gtt ttc agt cac agg tct aag tcc att     240
   gac atg ata att agt cac ctg tct ctc atc cac ata ctg ctg ctc ttc     288
   act cag gca ata ttg gtg tcc tta gac ttc ttt ggt tca cag aat act     336
   cag gat gat ctt agg tat aag gtc att gtc ttt tta aac aag gtg atg     384
   agg ggc ctc tcc atc tgc acc ccc tgc ctc ctg agt gtg ctc cag gcc     432
   atc atc agc ccc agc atc ttc tcc ttg gca aag ctc aaa cat cct tct     480
   gca agt cac atc tta gga ttc ttc ctt ttc tca tgg gtc ctc aac atg     528
   ttc att ggt gta atc ttc tgc tgt aca ctg cgg cta ccc cca gtg aaa     576
   cgg ggc cag tct tct gtt tgt cat aca gca ctg ttc ctt ttt gcc cat     624
   gag cta cac cca cag gag act gtt ttt cac act aat gac ttt gag gga     672
   tgt cac ctt tat agg gtt cat ggt cct ctc aag agg cta cat ggt gat     720
   tat ttt ata cag aca ata aga ggc tat ctc agt gcc ttc aca cag cca        768
   gcc tgt ccc cga gtc tca cca gtg aaa aga gcc tcc cag gct atc tta        816
   ctg ctg gtg agt ttt gtc ttc aca tac tgg gtg gac ttt acg ttc tca        864
   ttt tca gga ggt gtg aca tgg ata aat gat tct ctg cta gtg tgg ctc        912
   cag gtt att gtg gcc aat agc tat gcc gca att agt cct ttg atg cta        960
   att tat gct gat aac caa ata ttc aag act ctg caa atg tta tgg ttt        1008
   aaa tat ttg tct cct cca aag ctc atg ttg aaa ttt aat cgc caa tgt        1056
   ggc agt act aag aag tga                                                1074
<210> 4
   <211> 357
   <212> PRT
   <213> Homo Sapiens
<400> 4